(19) **Europäisches Patentamt**

**European Patent Office**

**Office européen des brevets**

(11) **EP 0 892 627 B1**

(12) **EUROPÄISCHE PATENTSCHRIFT**

(45) Veröffentlichungstag und Bekanntmachung des Hinweises auf die Patenterteilung:
**20.08.2003 Patentblatt 2003/34**

(21) Anmeldenummer: **97903208.3**

(22) Anmeldetag: **06.03.1997**

(51) Int Cl.⁷: $A61F\ 2/32$

(86) Internationale Anmeldenummer:
**PCT/CH97/00090**

(87) Internationale Veröffentlichungsnummer:
**WO 97/038650 (23.10.1997 Gazette 1997/45)**

(54) **KÜNSTLICHES GELENK**

ARTIFICIAL JOINT

ARTICULATION ARTIFICIELLE

(84) Benannte Vertragsstaaten:
**AT BE CH DE ES FR GB IE IT LI LU NL SE**

(30) Priorität: **12.04.1996 EP 96810225**

(43) Veröffentlichungstag der Anmeldung:
**27.01.1999 Patentblatt 1999/04**

(60) Teilanmeldung:
**03007042.9**

(73) Patentinhaber: **Centerpulse Orthopedics Ltd.
6340 Baar (CH)**

(72) Erfinder: **SEMLITSCH, Manfred
CH-8400 Winterthur (CH)**

(74) Vertreter: **Manitz, Finsterwald & Partner Gbr
Postfach 31 02 20
80102 München (DE)**

(56) Entgegenhaltungen:
**EP-A- 0 226 762        EP-A- 0 554 214
EP-A- 0 578 322        EP-A- 0 648 478
WO-A-95/23566**

• **BIOMEDIZINISCHE TECHNIK, Bd. 35, Nr. 5, 1990, BERLIN DE, XP000159817 STREICHER ET AL.: "UNTERSUCHUNG DES TRIBOLOGISCHEN VERHALTENS VON METALL/METALL-KOMBINATIONEN FÜR KÜNSTLICHE HÜFTGELENKE"**

**Beschreibung**

Die Erfindung bezieht sich auf ein künstliches Gelenk, insbesondere ein künstliches Hüftgelenk, mit den Merkmalen des Anspruchs 1.

Künstliche Gelenke erfordern Materialpaarungen der sich gegeneinander bewegenden Lagerkörper, die gute Notlaufeigenschaften aufweisen. Der klassische Ansatz in der Materialkombination besteht daher auch in der Paarung ungleicher Partner. So werden relativ weiche Lagerschalen aus Polyäthylen mit harten Gelenkköpfen aus Metall oder Keramik kombiniert und in den Anfängen der künstlichen Hüftgelenke metallische Werkstoffe mit unterschiedlicher Härte und Verschleissfestigkeit miteinander kombiniert. Trotz aller Anstrengungen konnte mit diesen Werkstoffkombinationen der Verschleiss der Partner nie ganz eliminiert werden. Bei Polyäthylen findet beispielsweise im Hüftgelenk ein Abrieb statt, durch welchen die Lagerfläche jährlich um ca. 0,2 mm in Richtung der Hauptkraft zurückgesetzt wird und auch bei metallischen Flächen treten durch Punktlasten und Mikroverschweissungen in den Oberflächen Verschleisserscheinungen auf, die - wenn sie einmal angefangen haben - sehr schnell die ganze Eingriffsfläche in Mitleidenschaft ziehen.

In der Publikation STREICHER: "Untersuchung des tribologischen Verhaltens von Metall/Metall Kombinationen für künstliche Hüftgelenke" (BIOMEDIZINI-SCHE TECHNIK, Bd. 35, Nr. 5, 1990, Berlin, DE, S. 107-111) werden Versuchsanordnungen beschrieben, die eine recht genaue Verschleissmessung bei Verschleissversuchen an künstlichen Hüftgelenken erlauben. Untersucht wurde der Verschleiss zwischen gleichartigen CoCrMo-Legierungen und CoCrMo-Legierung gegen UHMWPE als Referenz. Es wurden Paarungen mit unterschiedlichem Spiel zwischen Kugel und Pfanne untersucht. Die Auswertung der Versuche erfolgte durch permanente Aufnahme des Reibungsmomentes sowie durch periodisches Ausmessen der Implantatskomponenten. Ausserdem wurde die Methode der Dünnschichtaktivierung angewendet, um auch kleine Verschleissraten feststellen zu können. Die Auswertung zeigt nur eine geringe Abhängigkeit der Reibung von der Gleitgeschwindigkeit, sowie ein höheres Reibmoment der CoCrMo-Gusslegierung gegen sich selbst im Vergleich gegen Polyethylen. Eine Reduktion des Lagerdurchmessers in den Bereich eines Lagers mit Polymer/Metallpaarung ergab für die Paarung von CoCr-Mo-Schmiedelegierungen ähnliche Reibungsverluste wie bei der Polymer/Metallpaarung. Für den Verschleiss wird bei gleichem Spiel der Einfluss des Lagerdurchmessers aufgezeigt. Ausserdem wird gezeigt, dass das Reibmoment bei einer Metall/Metall Paarung nach einer Einlaufphase mit höherem Reibmoment zurückgehen kann. Nicht gezeigt ist, welche Anfangsgeometrie notwendig ist, um zu verschleissarmen Paarungen zu kommen, noch sind Verfahren aufgezeigt, die zu einer Verbesserung der Situation führen.

Ein künstliches Gelenk mit metallischen Lagerflächen, das bestimmten geometrischen Bedingungen gemigt, ist aus der WO 97/16138 A1 bekannt. Dieses Dokument fällt unter Art. 54(3) EPÜ.

Aufgabe der Erfindung ist es, ein möglichst verschleissarmes künstliches Gelenk sowie ein Verfahren zur Herstellung eines derartigen Gelenks zu schaffen.

Die Lösung dieser Aufgabe erfolgt zum einen durch die Merkmale des unabhängigen Vorrichtungsanspruchs 1, wonach Lagerschale und Gelenkkopf aus einem verschleissfesten metallischen Werkstoff bestehen, die Fläche A einen mittleren Radius $R_m$ und die Fläche B einen mittleren Radius $r_m$ aufweisen, wobei deren Differenz 35 μm < $R_m$-$r_m$ < 85 μm beträgt, der Formfehler der Fläche A über einen Winkel 90° < α < 180° weniger als plus/minus 7,5 μm beträgt, der Formfehler der Fläche B über einen Winkel β > 140° weniger als plus/minus 2 μm beträgt, und die Gelenkkugel ausserhalb der Fläche B durch eine zurückgesetzte Fläche C fortgesetzt ist, die einen geringeren Abstand zum Mittelpunkt $M_K$ als die Fläche B aufweist, während die Rauhigkeit der Fläche A einem Wert $R_a$ < 0,08 μm und die Rauhigkeit der Fläche B einem Wert $R_a$ < 0,08 μm entspricht. Durch das Herstellen, Vermessen und Paaren von Lagerflächen aus einem gleichartigen verschleissfesten, metallischen Werstoff wird zwischen den Lagerflächen eine Geometrie erreicht, die zusammen mit der Kapillarwirkung der Körperflüssigkeit und dem Auftrieb bei aneinander vorbeigleitenden Lagerflächen Mikroverschweissungen und Verschleiss weitgehend verhindert. Durch das Unterdrücken der Mikroverschweissungen können bei gleichartigen, verschleissfesten metallischen Werkstoffen, die positiven Eigenschaften dieser Werkstoffe wie Zähfestigkeit, Formbeständigkeit und Elastizität genutzt werden. Es entstehen Oberflächen an einem homogenen Gefüge, die weder wegen Härteunterschieden zwischen Oberfläche und Grundkörper bei starker Belastung durchbrochen werden noch sich vom Grundkörper ablösen. Gleichzeitig sind die Flächen so gut aneinander angeglichen, dass keine unzulässige Pressung im Stillstand auftritt.

Diese Wirkung wird noch besser, wenn die Rauhigkeit der Flächen A und B einem Wert $R_a$ < 0,05 μm entspricht.

Besonders geeignet sind Werkstoffe wie Kobalt-, Chrom-, Nickellegierungen wie beispielsweise nach ISO 5832/4 das Material PROTASUL 21 WF der Firma SULZER AG, wenn sie nach einem Verfahren gefertigt werden, bei dem eine kreisförmige Erzeugende für die Kugelform ebenfalls dreht, jedoch in ihrer Drehachse zur Drehachse des Werkstücks verschwenkt ist, um eine Kugelform zu schleifen, zu honen und zu polieren bis die vorgegebenen Toleranzen für Durchmesser, Formgenauigkeit und Oberflächengüte erreicht sind.

Vorteilhafte Weiterbildungen der Erfindung ergeben sich aus den abhängigen Ansprüchen 2 bis 5 sowie 13 bis 15. Lösbare Verbindungen für Innenschalen sind durch die Verwendung von Innenschalen aus relativ elastischem Polyäthylen bekannt. Diese lassen sich nicht von Innenschalen aus wesentlich zähfesteren metallischen Werkstoffen übernehmen. Funktion und Herstellung sprechen dagegen. Es ist daher sinnvoll, die Lagerschale an ihrer Aussenseite fest mit einem Zwischenkörper aus elastischem Kunststoff wie beispielsweise Polyäthylen zu verbinden, der seinerseits mit einer äusseren Schale lösbar verbunden werden kann. Insbesondere können auch implantierte Kunststoffschalen durch die metallische Lagerschale ersetzt werden, wenn die lösbare Verbindung des Zwischenkörpers die gleiche ist, und ebenso können die von der Präzision her notwendigen Gelenkkugeln an Prothesenschäften ausgewechselt werden, wenn die Gelenkkugeln über eine lösbare Verbindung, zum Beispiel über eine lösbare Konusverbindung, auf dem Schaft verfügen. Dadurch, dass Gelenkkopf und Lagerschale auswechselbar gestaltet sind, können sie steril verpackt bis auf den Operationstisch gebracht werden. Die Lagekontrolle eines implantierten Gelenks erfolgt noch mit Manipuliergelenkkugeln, die die Schalen nicht verletzen, während die Präzisionskugeln erst gegen Schluss eingesetzt werden.

Interessanterweise hat sich gezeigt, dass für eine verschleissarme Paarung Metall/Metall bei vorgegebener Rauhigkeit Ra die Formfehler der einzelnen Teile für die Schmierung eine grössere Rolle spielen als die Bandbreite, innerhalb welcher die Differenz der mittleren Radien $R_m$-$r_m$ liegen darf. Bei einem für künstliche Hüftgelenke üblichen Kugeldurchmesser beispielsweise für einen Nenndurchmesser von 28 mm darf die Differenz der mittleren Radien 35 µm < $R_m$-$r_m$ < 85 µm betragen, was einer Bandbreite von 50 µm entspricht, wenn man von absoluten angestrebten Herstellmassen und nicht von Auswahlpaarungen ausgeht. Bei einer Halbierung dieses Bandes würde für beide Teile eine absolute Herstellgenauigkeit von 25 µm für den mittleren Radius $R_m$ oder $r_m$ verbleiben. Diese Werte sind gross genug, um auf eine Auswahlpaarung verzichten zu können und somit jede Kugel mit jeder Lagerschale paaren zu können. Dies ist jedoch nur möglich, wenn die Formgenauigkeit beherrscht wird. Diese ist sehr genau einzuhalten und bedarf besonderer Herstellmethoden, um die erforderlichen Toleranzwerte einzuhalten.

Die Lösung der der Erfindung zugrunde liegenden Aufgabe erfolgt zum anderen durch die Merkmale des unabhängigen Verfahrensanspruchs 6, wonach die vorgegebenen Werte für Durchmesser, Formgenauigkeit und Rauhigkeit dadurch erreicht werden, dass die Lagerschale als Werkstück als eine mit Untermass im Bereich der Lagerfläche A vorgedrehte Schale mit ihrer Polachse in der Rotationsachse einer Werkzeugmaschinenspindel aufgespannt wird oder dass die vorgedrehte Gelenkkugel als Werkstück mit Uebermass im Bereich der Lagerfläche B mit ihrer Aufspannachse D in der Rotationsachse einer Werkzeugmaschinenspindel aufgespannt wird und dass während dem Rotieren des Werkstücks ein kreiszylindrischer Schleifkörper, der mit seiner Zylinderachse in der Rotationsachse einer Werkzeugspindel rotierend aufgespannt ist, mit einer kreisförmigen Kante seiner Stirnseite unter Zugabe von Schleifmittel an die Lagerfläche A, B des Werkstücks angepresst wird, wobei sich die Rotationsachse der Werkzeugspindel in einem Auslenkwinkel $\gamma$, $\delta$ kleiner als 90° mit der Rotationsachse der Werkstückspindel in einem Schnittpunkt schneidet und wobei der Anpressdruck durch Zustellen der Werkzeugspindel in der Richtung ihrer Rotationsachse erfolgt.

Diese Anordnung hat den Vorteil, dass sich Werkzeug und Werkstück - im Rahmen der Steifigkeit der Spindeln an denen sie eingespannt sind - an den Arbeitsflächen gegenseitig zentrieren. Aufgrund der Ablaufbewegung entsteht an Werkstück und an Werkzeug ein Verschleiss der zwangsläufig zur Bildung einer Kugelfläche an beiden Teilen führt. Am Werkzeug entsteht an einer gebrochenen Kante an der Stirnseite ein schmales kreisförmiges Band einer Kugelfläche während am Werkstück für die gleiche Kugelform Kugelflächen A, B entstehen. Dadurch, dass jeder Punkt der Werkzeugarbeitsfläche gegenüber jedem Punkt der Bearbeitungsfläche in Eingriff kommt entstehen perfekte Ausschnitte A, B. von Kugelflächen.

Die abhängigen Ansprüche 7 bis 12 stellen vorteilhafte Weiterbildungen des Verfahrens dar. So ist es für die Herstellung der Lagerfläche A einer Lagerschale vorteilhaft, den Winkel zwischen Rotationsachsen von Lagerschale und Werkzeug so zwischen 39° und 45° zu wählen, dass ein möglichst grosser Begrenzungswinkel $\alpha$ für die Lagerfläche entsteht, weil dann der Durchmesser für die Erzeugende und einen ihr entsprechenden Zylinder so gross gewählt werden kann, dass der Begrenzungswinkel $\alpha$ gegen 180° wachsen kann, ohne dass dieser Zylinder den Schaleninnnenrand touchiert. Es ist somit nur eine geführte Zustellbewegung in der Richtung der Rotationsachse des Werkzeugs notwendig, um zu einem grossen Begrenzungswinkel $\alpha$ zu kommen. Bei einem Begrenzungswinkel $\alpha$ der um einen beträchtlichen Betrag kleiner als 180° ist, können zwar grössere Kreisdurchmesser für eine Erzeugende gewählt werden, hingegen ist die Erzeugende dann nur noch als unterbrochener Kreis im Eingriff.

Bei der Herstellung der Gelenkkugel hat sich gezeigt, dass eigentlich schon eine Lagerfläche B mit einem Begrenzungswinkel $\beta$ von etwa 180° bei einer Formgenauigkeit von plus/minus 2 µm für die Funktion des Lagers genügt, sofern einerseits alle anderen Flächenteile an der Gelenkkugel weiter zurückstehen und andererseits im Zustand der Normalbelastung der Äquator von Lagerschale und von Lagerfläche B annähernd parallel zueinander ausgerichtet sind. Der Auslenkwinkel $\gamma$ für ein rotierendes Werkzeug mit einem kreiszylindrischen Hohlzylinder kann in grösseren Grenzen.beispielsweise zwischen 60° bis 20° eingestellt werden, um eine Lagerfläche B zu erzeugen. Da die Erzeugende Kreisringfläche auch bei einem Begrenzungswinkel β von mehr als 180° nicht bis an den Mittelpunkt der Lagerfläche B herankommt, kann der Durchmesser der Gelenkkugel über die Lagerfläche B während der Bearbeitung, beispielsweise durch einen Taster mit diamantbestückten Abtastflächen, abgefragt werden, um die restliche Bearbeitungszeit genügend genau zu extrapolieren.

Abgesehen vom Polieren eines Einlaufradius am Äquator der Lagerfläche A der Lagerschale können die anderen Arbeitsgänge für die erforderliche Präzision von Lagerschale und Gelenkkugel auf einer numerisch gesteuerten Werkzeugmaschine automatisch durchgeführt werden.

Der geringe Verschleiss an Kugel und Lagerschale eröffnet im weiteren den Vorteil, dass mit der erfindungsgemässen Ausführung keine Reoperation wegen sich verschlechternder Lagerflächen notwendig ist. Aus diesem Grund können die Gelenkkugeln bei zementierten Prothesenschäften sogar einstückig mit dem Schaft verbunden sein und bei direkt eingesetzten Schäften, z.B. aus Titan, mit einer Einmal-Verbindung befestigt werden, solange die Operationstechnik nicht aus Platzgründen eine nachträgliche Befestigung der Kugel am implantierten Schaft vorschreibt. Insbesondere lohnt es sich anatomisch angeglichene und fest verankerbare Schäfte, die eine S-förmige Schaftform mit einer Anteversion vom proximalen Halsbereich und mit einem gegen posterior in einer Krümmung oder in einem Knick wegstehenden Schaftende besitzen, einzusetzen, da nur noch die Dauer der Verankerung den Termin für einen weiteren Eingriff bestimmt.

Im folgenden wird die Erfindung anhand von Ausführungsbeispielen beschrieben. Es zeigen:

Fig. 1 Schematisch einen Längsschnitt durch eine Lagerschale;

Fig. 2a schematisch einen Schnitt durch eine Gelenkkugel mit einem Begrenzungswinkel β > 180°;

Fig. 2b schematisch einen Schnitt durch eine Gelenkkugel mit einem Begrenzungswinkel β < 180°;

Fig. 3 schematisch und abgewickelt den Profilschrieb einer Lagerschale und den einer Gelenkkugel, die im Abstand ihrer mittleren Radien zueinander angeordnet sind;

Fig. 4 schematisch eine Anordnung von Gelenkkugel und Werkzeug bei der Herstellung auf einer Werkzeugmaschine;

Fig. 5 schematisch eine Anordnung von Lagerschale und Werkzeug bei der Herstellung auf einer Werkzeugmaschine; und

Fig. 6 schematisch die Ausführung einer Lagerschale, welche mit einem Kunststoffzwischenkörper fest verbunden ist, der seinerseits lösbar in einer äusseren Schale befestigt ist.

Mit den Figuren wird eine Geometrie beschrieben, die es ermöglicht, gleichartige, verschleissfeste, metallische Werkstoffe wie PROTASUL 21 WF als Lagerschale 1 und Gelenkkugel 2 in einem sphärischen Lager zu verwenden, ohne dass Mikroverschweissungen und exzessiver Verschleiss auftreten, während andere Eigenschaften wie Zähfestigkeit, Formbeständigkeit und Elastizität für die Funktion genutzt werden können. Durch eine passende Relation zwischen den mittleren Radien $R_m$, $r_m$ der Lagerflächen A und B sowie der zulässigen Formabweichungen 12, 13 und der zulässigen Rauhigkeit der Lagerflächen werden Mikroverschweissungen des gleichartigen metallischen Werkstoffs weitgehend verhindert.

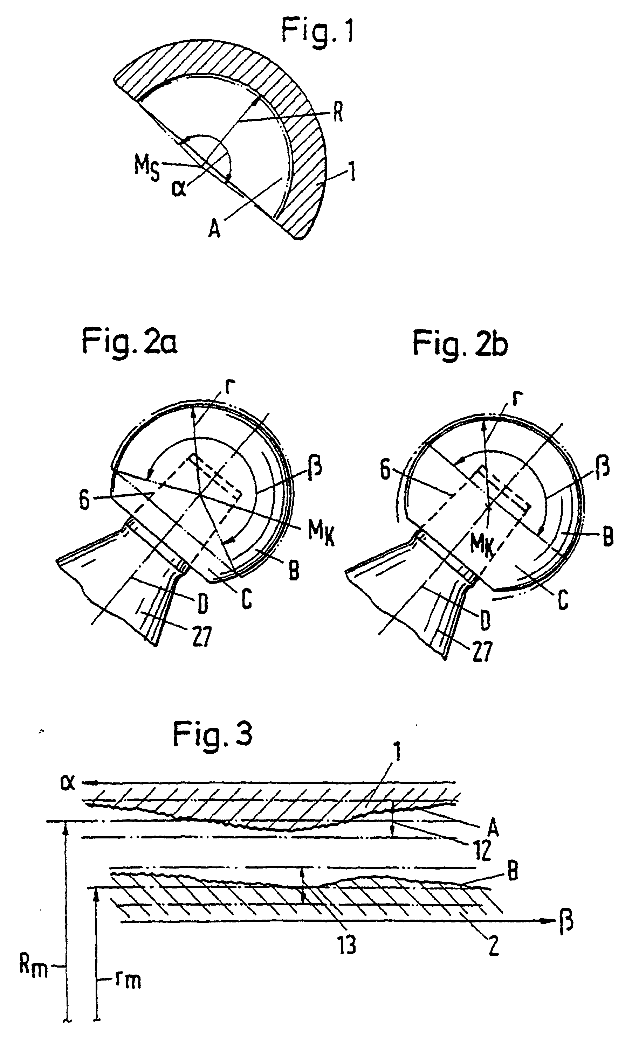

In Figur 1 ist eine Lagerschale 1 für ein künstliches Hüftgelenk dargestellt, deren sphärische Lagerfläche A sich über einen Winkel α von etwas weniger als 180° erstreckt und einen Abstand R zu einem Mittelpunkt der Fläche aufweist. Analog dazu ist in den Figuren 2a und 2b eine Gelenkkugel 2 mit einer sphärischen Lagerfläche B dargestellt, die sich über einen Winkel β > 140° erstreckt und die einen Abstand r zum Mittelpunkt $M_K$ der Gelenkkugel aufweist.

Beide Gelenkkugeln in Figur 2a und 2b sind ausserhalb der Lagerfläche B durch eine Quasikugelfläche C fortgesetzt, die jedoch einen geringeren Abstand als den Radius r der Lagerfläche B zum Mittelpunkt $M_K$ der Lagerfläche B aufweist. Der kleinere Abstand entsteht beispielsweise dadurch, dass der Gelenkkopf vor dem Schleifen im Hinblick auf den späteren Mittelpunkt $M_K$ im Bereich C bereits abgeplattet oder kegelförmig ausgebildet ist. Die Gelenkkugel ist über eine lösbare Konusverbindung 6 mit einem Prothesenschaft 27 verbunden, wobei die Aufspannachse D vom Konus mit der Rotationsachse für die rotationssymmetrische Lagerfläche B zusammenfällt, damit die Lagerfläche B unabhängig von der konischen Befestigung immer die gleiche Lage einnimmt.

In Figur 3 ist für die sphärischen Flächen A und B jeweils in abgewickelter Form ein Tastschnitt über die Oberfläche dargestellt. Ausgehend von einer nicht gezeigten gemeinsamen Grundlinie ist in einem mittleren Abstand $R_m$ die Fläche A über einen Winkel α und in einem mittleren Abstand $r_m$ die Fläche B über einen Winkel β aufgezeichnet. Dabei ist die Vergrösserung senkrecht zur Abtastrichtung, um mehrere Zehnerpotenzen grösser als die Vergrösserung in Abtastrichtung dargestellt. Der zulässige Formfehler für die Fläche A der Lagerschale zum mittleren Radius $R_m$ liegt in einer Bandbreite von plus/minus 7,5 μm und die Rauhigkeit beträgt $R_a < 0,05$ μm. Für die Fläche B der Kugel beträgt der zulässige Formfehler zum mittleren Radius $r_m$ plus/minus 2 μm und die Rauhigkeit $R_a$ < 0,05 μm. Wenn zu dieser Kombination die Differenz der mittleren Radien in den Grenzen 35 μm < $R_m$-$r_m$ < 85 μm liegt und als Werkstoff für die Lagerschale 1 und den Gelenkkopf 2 eine verschleissfeste Metall-Legierung vorliegt, die beispielsweise Blockkarbide als Stützkörper eingelagert hat, dann werden im Beisein von Körperflüssigkeit Tragzahlen erreicht, die trotz der gleichartigen Metall-Legierungen Mikroverschweissungen und Zerstörungen der Oberflächen für die normalen Belastungen an einem Hüftgelenk weitgehend ausschliessen.

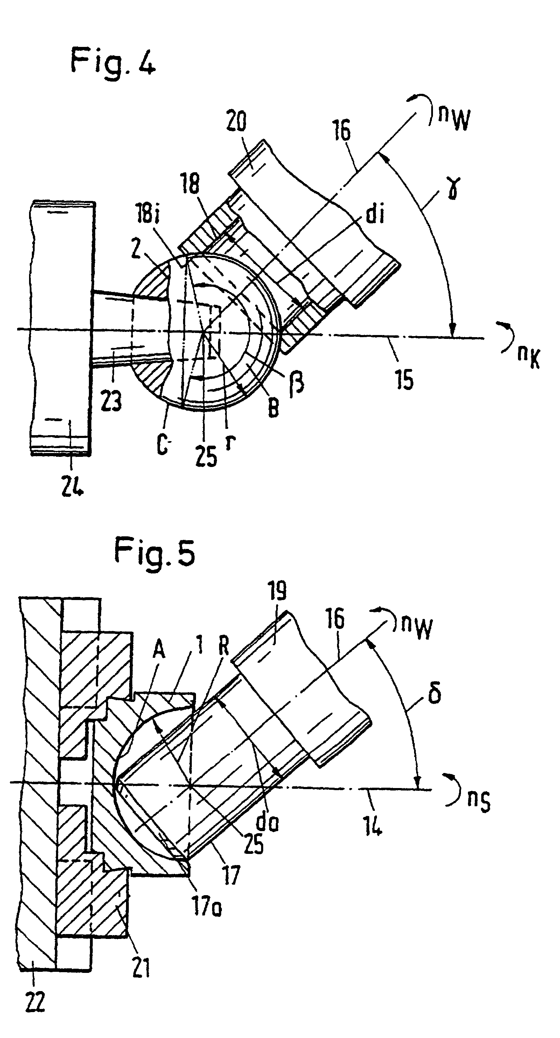

Die Anordnung in Figur 4 betrifft eine Gelenkkugel 2, die mit einem Innenkonus auf einem Aufspanndorn 23 befestigt ist, wobei der Dorn 23 zu einer Werkzeugmaschinenspindel 24 gehört und mit einer Drehzahl $n_K$ von beispielsweise 850 U/min. um seine Rotationsachse 15 dreht. Eine um einen Winkel γ von beispielsweise 30° ausgelenkte Werkzeugspindel 20 dreht um ihre Rotationsachse 16 mit einer Drehzahl von beispielsweise 2 000 U/min., wobei sich die beiden Rotationsachsen 15, 16 in einem Schnittpunkt 25 schneiden, der den Mittelpunkt für die später fertig bearbeitete Gelenkkugel 2 bildet. In der Werkzeugspindel 20 ist koaxial ein kreisförmiger Hohlzylinder 18 als Bearbeitungswerkzeug zum Schleifen, Honen oder Polieren eingespannt, welcher mit einer kreisförmigen gebrochenen Innenkante 18i eine Erzeugende mit einem Innendurchmesser di bildet. Der Hohlzylinder besteht aus den üblichen Werkstoffen für Schleifen ohne gebundenes Korn wie z.B. aus Metalloxiden oder Karbiden. Durch Zusetzen eines nicht gezeigten Schleifmittels und Anpressen der Stirnseite des Hohlzylinders 18 in der Richtung der Vorschubund Rotationsachse 16 schleifen sich die erzeugende Fläche 18i und die Lagerfläche B gegenseitig zu perfekten Ausschnitten von Kugelflächen ein, wobei sich der Radius r der Lagerfläche B ganz langsam verringert und die Erzeugende 18i zu einem kreisförmigen, kugelförmig gewölbten Band vergrössert wird. Durch entsprechende Materialwähl kann der Verschleiss an diesem Band klein gehalten werden. Die so entstehende Lagerfläche B lässt sich durch einen zugehörigen Begrenzungswinkel β definieren und hängt in ihrer Grösse vom Auslenkwinkel γ des Werkzeugs und vom Durchmesser di der Erzeugenden ab. Dementsprechend sind Lagerflächen B mit einem Begrenzungswinkel β grösser und kleiner 180° möglich, wie in den Figuren 2a, 2b angedeutet ist. Bei einem Begrenzungswinkel β > 180°, wie in Figur 4, kann die Abnahme vom Radius r während dem Schleifen durch eine Durchmessermessung über den Schnittpunkt 25 der beiden Rotationsachsen 15, 16 festgestellt werden, um den Abbruch des Schleifens für das Fertigmass vom Radius r über eine extrapolierbare Zeit festzulegen. Für den Auslenkwinkel γ und für den Innendurchmesser di lässt sich als Vorzugsbereich

$20° \leq γ \leq 60°$ und $1,8\ r > d_i > 1,1\ r$ angeben.

In der Anordnung nach Figur 5 ist eine Lagerschale 1 in einer Werkzeugmaschinenspindel 22 mit einem Spannfutter 21 derart eingespannt, dass die Polachse der Lagerschale 1 und die Rotationsachse 14 der Spindel 22 zusammenfallen, wobei die Spindel 22 mit einer Drehzahl $n_s$ von beispielsweise 850 U/min. dreht. Eine Werkzeugspindel 19 ist um einen Auslenkwinkel δ ausgelenkt und schneidet mit ihrer Rotationsachse 16 die Rotationsachse 14 der Werkstückspindel 22 in einem Schnittpunkt 25, der dem Mittelpunkt der späteren Lagerfläche A entspricht. In die Werkzeugspindel ist koaxial als Werkzeug zum Schleifen ohne gebundenes Korn ein kreisförmiger Vollzylinder 17 eingespannt der mit einer Drehzahl von beispielsweise 2 050 U/min. dreht und mit einer Aussenkante seiner Stirnfläche eine kreisförmige Erzeugende 17a bildet. Durch Zugabe von Schleifmittel und Nachstellen der Erzeugenden 17 in der Richtung der Rotationsachse 16 des Werkzeugs schleifen sich Lagerfläche A und Erzeugende 17a zwangsläufig zu perfekten Ausschnitten von Kugelflächen ein. Um das Mass für den Radius R besser in Griff zu bekommen, wird die Kante 17 vorsorglich zu einer Kugelform abgerichtet. Dies hat den Vorteil, dass der Verschleiss am Werkzeug nur geringfügige Massänderungen verursacht und dass das Einhalten von einem vorgegebenen Radius R an der Lagerschale 1 erleichtert wird. Für den Auslenkwinkel δ ergibt sich ein Vorzugsbereich 39° < δ < 45°. Wenn der Begrenzungswinkel α für die Lagerfläche nicht zu weit unter 180° liegen soll und zum Beispiel nur ein Vorschub in der Richtung der Werkzeugrotationsachse 16 erfolgen soll, ergibt sich für den Aussendurchmesser $d_a$ der Erzeugenden und den Auslenkwinkel δ ein bevorzugter Anwendungsbereich

$$1,6\ R < \frac{d_a}{\cos δ} < 2,2\ R.$$

Die Darstellung in Figur 3 dient dazu, die prinzipiellen Zusammenhänge über die Massunterschiede der mittleren Radien $R_m$, $r_m$ sowie über die Toleranz 12 für den Formfehler der Lagerschale und die Toleranz 13 für den Formfehler der Gelenkkugel und über die Rauhigkeit der Flächen A, B aufzuzeigen. In der Praxis wird die Qualität der Gelenkkugeln 2 durch Rundheitsmessungen mit einem "Talyround"-Messgerät überwacht, während die Lagerschalen auf einer Messmaschine auf ihrer Innenseite in verschiedenen Ebenen radial mit einem Messfühler angefahren werden, um aus den Messpunkten eine mittlere Kugelform zu bestimmen und die Formabweichungen zu interpolieren. Der zulässige Formfehler für eine Gelenkkugel 2 im Bereich der Lagerfläche B beträgt plus/minus 2 μm, während für die Lagerfläche A der Lagerschale ein Formfehler von plus/minus 7,5 μm zulässig ist. Bei beiden Teilen liegt die Rauhigkeit $R_a$ unter 0,08 μm, vorzugsweise unter 0,05 μm.

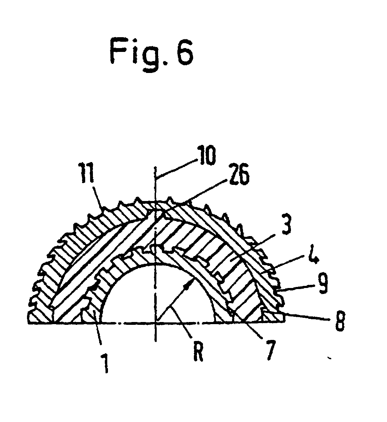

Die Tragfähigkeit dieser fast verschleissfreien Anordnung ist so günstig, dass der Radius für den Gelenkkopf und für die Lagerschale, wie in Figur 6 angedeutet, kleiner als der einer üblichen Polyäthylenlagerschale ausgeführt werden kann. Das heisst, das Auswechseln von Lagerschale und Gelenkkopf ist auch bei implantierten äusseren Schalen 4 möglich, die eine auswechselbare Polyäthylenschale aufweisen, weil die metallische Lagerschale 1 mit einem Zwischenkörper 3 von ausreichender Wandstärke aus Polyäthylen versehen werden kann, dessen äussere Abmessungen denen der ursprünglichen Polyäthylenschale entsprechen. Dabei kann der fest mit der Lagerschale 1 über eine Verbindung 7 verbundene Zwischenkörper 3 eine etwas geringere Wandstärke als die ursprüngliche Polyäthylenlagerschale aufweisen. Dies hat den Vorteil, dass implantierte auswechselbare Polyäthylenschalen durch eine entsprechende Metallschale mit Polyäthylenzwischenkörper ersetzt werden können. Die Aussenschale 4 in Figur 6 besitzt bis auf zwei Drittel der Höhe ihrer Aussenseite eine Zahnung 9, die zum Äquator deutet, während im oberen Drittel der Aussenseite Spitzen 11 angebracht sind, welche parallel zur Polachse 10 ausgerichtet sind. Eine solche Aussenschale ist in eine mit Untermass vorbereitete Knochenaushöhlung einschlagbar. Dabei können die Spitzen 11 in ein nicht gezeigtes Knochenbett eindringen, während die abwärts gerichtete Zahnung am Knochenbett unter Vorspannung vorbeigleiten kann und in einer Endlage ein Rückwärtsgleiten mit den Zahnspitzen verhindert, um eine ausreichende Primärverankerung zu erreichen.

**Patentansprüche**

1. Künstliches Gelenk mit einer Lagerschale (1), die eine konkave sphärische Fläche A mit Mittelpunkt $M_S$ aufweist, und mit einer Gelenkkugel (2), die eine konvexe sphärische Fläche B mit Mittelpunkt $M_K$ aufweist, wobei

   Lagerschale (1) und Gelenkkopf (2) aus einem verschleissfesten metallischen Werkstoff bestehen,

   die Fläche A einen mittleren Radius $R_m$ und die Fläche B einen mittleren Radius $r_m$ aufweisen, wobei deren Differenz $35 \, \mu m < R_m\text{-}r_m < 85 \, \mu m$ beträgt,

   der Formfehler der Fläche A über einen Winkel $90° < \alpha < 180°$ weniger als plus/minus $7{,}5 \, \mu m$ beträgt,

   der Formfehler der Fläche B über einen Winkel $\beta > 140°$ weniger als plus/minus $2 \, \mu m$ beträgt, und

   die Gelenkkugel (2) ausserhalb der Fläche B durch eine zurückgesetzte Fläche C fortgesetzt ist, die einen geringeren Abstand zum Mittelpunkt $M_K$ als die Fläche B aufweist, während die Rauhigkeit der Fläche A einem Wert $R_a < 0{,}08 \, \mu m$ und die Rauhigkeit der Fläche B einem Wert $R_a < 0{,}08 \, \mu m$ entspricht.

2. Künstliches Gelenk nach Anspruch 1, **dadurch gekennzeichnet, dass** die Rauhigkeit der Fläche A einen Wert $R_a < 0{,}05 \, \mu m$ und die Rauhigkeit der Fläche B einen Wert $R_a < 0{,}05 \, \mu m$ aufweist.

3. Künstliches Gelenk nach Anspruch 1 oder 2, **dadurch gekennzeichnet, dass** die Lagerschale (1) auf ihrer Aussenseite mit einem Zwischenkörper (3) über eine Verbindung (7) fest verbunden ist, wobei der Zwischenkörper mit einer äusseren Schale (4) lösbar verbindbar ist.

4. Künstliches Gelenk nach Anspruch 3, **dadurch gekennzeichnet, dass** das Material des Zwischenkörpers um mehr als einen Faktor 10 elastischer ist als der Werkstoff der metallischen Lagerschale.

5. Künstliches Gelenk nach Anspruch 1, **dadurch gekennzeichnet, dass** die Gelenkkugel über eine lösbare Konusverbindung (6) in der Richtung der Aufspannachse D mit dem Schaft verbindbar ist, um unabhängig von der Verbindung eine gleich wirkende Teilkugelfläche B zu erhalten.

6. Verfahren zum Herstellen eines künstlichen Gelenks nach Anspruch 1, bei welchem

   die Lagerschale (1) als Werkstück als eine mit Untermass im Bereich der Lagerfläche A vorgedrehte Schale mit ihrer Polachse (10) in der Rotationsachse (14) einer Werkzeugmaschinenspindel (22) aufgespannt wird oder

   die vorgedrehte Gelenkkugel (2) als Werkstück mit Uebermass im Bereich der Lagerfläche B mit ihrer Aufspannachse D in der Rotationsachse (15) einer Werkzeugmaschinenspindel aufgespannt wird, und

   während dem Rotieren des Werkstücks ein kreiszylindrischer Schleifkörper (17, 18), der mit seiner Zylinderachse in der Rotationsachse (16) einer Werkzeugspindel (19, 20) rotierend aufgespannt ist, mit einer kreisförmigen Kante (17a, 18) seiner Stirnseite unter Zugabe von Schleifmittel an die Lagerfläche (A, B) des Werkstücks (1, 2) angepresst wird,

   wobei sich die Rotationsachse (16) der Werkzeugspindel (19, 20) in einem Auslenkwinkel $(\gamma, \delta)$ $< 90°$ mit der Rotationsachse der Werkstückspindel (14, 15) in einem Schnittpunkt (25) schneidet und

   wobei der Anpressdruck durch Zustellen der Werkzeugspindel (19, 20) in der Richtung ihrer Rotationsachse (16) erfolgt.

7. Verfahren nach Anspruch 6, **dadurch gekennzeichnet, dass** der Winkel $(\delta)$ zwischen der Rotationsachse (16) der Werkzeugspindel (19) und der Rotationsachse (14) der Lagerschalenspindel (22) zwischen 45° und 39° beträgt und dass der Aussendurchmesser $d_a$ von der kreisförmigen Kante (17a) so gewählt ist, dass

$$1{,}6 \ R < \frac{d_a}{\cos \delta} < 2{,}2 \ R$$

eingehalten ist.

8. Verfahren nach Anspruch 6, **dadurch gekennzeichnet, dass** der Winkel (γ) zwischen der Rotationsachse (16) der Werkzeugspindel (20) und der Rotationsachse (15) der Gelenkkugelspindel (24) zwischen 20° und 60° beträgt und dass der Innendurchmesser $d_i$ von der kreisförmigen Kante (18i) der Bedingung 1,8 r > $d_i$ > 1,1 r genügt.

9. Verfahren nach einem der Ansprüche 6 bis 8, **dadurch gekennzeichnet, dass** das Werkstück als Lagerschale (1) mit einer Drehzahl $n_S$ respektive als Gelenkkugel (2) mit einer Drehzahl $n_K$ dreht, während das Werkzeug (17, 18) eine annähernd doppelt so grosse Drehzahl $n_W$ wie das Werkstück aufweist, jedoch vorzugsweise kein ganzzahliges Vielfaches.der Werkstückdrehzahl bildet.

10. Verfahren nach einem der Ansprüche 6 bis 9, **dadurch gekennzeichnet, dass** es zum Schleifen, Honen oder Polieren verwendet wird.

11. Verfahren nach Anspruch 8, **dadurch gekennzeichnet, dass** während der Bearbeitung der Gelenkkugel (2) eine Durchmessermessung auf der Fläche B über den Mittelpunkt $M_K$ erfolgt, um aufgrund von gespeicherten Werten für die Abtragsleistung mit einer Steuerung eine Restzeit für die Bearbeitung auf einen vorgegebenen Durchmesser festzulegen.

12. Verfahren nach einem der Ansprüche 6 bis 11, **dadurch gekennzeichnet, dass** das Verfahren zum Herstellen eines künstlichen Hüftgelenks dient.

13. Künstliches Gelenk nach einem der Ansprüche 1 bis 5, **dadurch gekennzeichnet, dass** das künstliche Gelenk ein künstliches Hüftgelenk ist.

14. Künstliches Gelenk nach Anspruch 13, **dadurch gekennzeichnet, dass** der Schaft des künstlichen Hüftgelenks eine S-förmige, anatomisch angeglichene Schaftform mit einer Anteversion im proximalen Halsbereich und mit einem gegen posterior in einer Krümmung oder in einem Knick wegstehenden Schaftende aufweist, um eine möglichst lange Verankerungsdauer zu erreichen.

15. Künstliches Gelenk nach Anspruch 13 oder 14, **da-**

**durch gekennzeichnet, dass** die konvexe sphärische Fläche B rotationssymmetrisch zu einer Aufspannachse D in der Richtung des Schenkelhalses des Schaftes (27) des künstlichen Hüftgelenks angeordnet ist.

**Claims**

1. Artificial joint having a bearing shell (1) which has a concave spherical surface A with centre $M_S$, and a joint ball (2), which has a convex spherical surface B with centre $M_K$, wherein

   - the bearing shell (1) and the joint head (2) consist of a wear-resistant metallic material;
   - the surface A has an average radius $R_m$ and the surface B has an average radius $r_m$, with their difference amounting to 35 μm < $R_m$-$r_m$ < 85 μm;
   - the shape error of the surface A amounts to less than plus/minus 7.5 μm over an angle 90° < α < 180°;
   - the shape error of the surface B amounts to less than plus/minus 2 μm over an angle β > 140°; and
   - the joint ball (2) is continued outside the area B by a set-back surface C which has a smaller spacing to the centre $M_K$ than the surface B, whereas the roughness of the surface A corresponds to a value $R_a$ < 0.08 μm and the roughness of the surface B corresponds to a value $R_a$ < 0.08 μm.

2. Artificial joint in accordance with claim 1, **characterised in that** the roughness of the surface A has a value $R_a$ < 0.05 μm and the roughness of the surface B has a value $R_a$ < 0.05 μm.

3. Artificial joint in accordance with claim 1 or claim 2, **characterised in that** the bearing shell (1) is fixedly connected at its outer side to an intermediate body (3) via a connection (7), with the intermediate body being releasably connectable to an outer shell (4).

4. Artificial joint in accordance with claim 3, **characterised in that** the material of the intermediate body is more elastic by a factor of more than 10 than the material of the metallic bearing shell.

5. Artificial joint in accordance with claim 1, **characterised in that** the joint ball can be connected to the shaft via a releasable cone connection (6) in the direction of the mounting axis D in order to obtain a uniformly acting partial spherical surface B independently of the connection.

6. Method for the manufacture of an artificial joint in accordance with claim 1, in which

- the bearing shell (1) is mounted as a workpiece as a pre-turned shell with undersize in the region of the bearing surface A and with its polar axis (10) in the axis of rotation (14) of a machine-tool spindle (22); or
- the pre-turned joint ball (2) is mounted as a workpiece with oversize in the region of the bearing surface B with its mounting axis D in the axis of rotation (15) of a machine-tool spindle; and
- during the rotation of the workpiece, a circular edge (17a, 18) of a front face of a circularly cylindrical grinding body (17, 18), whose cylinder axis is rotatingly mounted in the axis of rotation (16) of a tool spindle, is pressed towards the bearing surface (A, B) of the workpiece (1, 2) while grinding means are added;
- with the axis of rotation (16) of the tool spindle (19, 20) intersecting the axis of rotation of the workpiece spindle (14, 15) at a point of intersection (25) and at a deflection angle ($\gamma$, $\delta$) < 90°; and
- with the contact pressure being exerted by advancing the tool spindle (19, 20) in the direction of its axis of rotation (16).

7. Method in accordance with claim 6, **characterised in that** the angle ($\delta$) between the axis of rotation (16) of the tool spindle (19) and the axis of rotation (14) of the bearing shell spindle (22) amounts to between 45° and 39°; and **in that** the outer diameter $d_a$ of the circular edge (17a) is chosen such that

$$1.6R < \frac{d_a}{\cos \delta} < 2.2R$$

is adhered to.

8. Method in accordance with claim 6, **characterised in that** the angle ($\gamma$) between the axis of rotation (16) of the tool spindle (20) and the axis of rotation (15) of the joint ball spindle (24) amounts to between 20° and 60°; and **in that** the inner diameter $d_i$ of the circular edge (18i) satisfies the condition 1.8 r > $d_i$ > 1.1 r.

9. Method in accordance with any one of claims 6 to 8, **characterised in that** the workpiece as a bearing shell (1) rotates with a rotating speed ns or, as a joint ball (2), with a rotating speed $n_K$, whereas the tool (17, 18) has a rotating speed nw which is approximately twice as large as the workpiece, but preferably does not form an integral multiple of the rotating speed of the workpiece.

10. Method in accordance with any one of claims 6 to 9, **characterised in that** it is used for grinding, honing or polishing.

11. Method in accordance with claim 8, **characterised in that**, during the machining of the joint ball (2), a diameter measurement is carried out over the surface B across the centre $M_K$ to establish, by using a control, a residual time for the machining to a predetermined diameter on the basis of stored values for the stock removal.

12. A method in accordance with any one of claims 6 to 11, **characterised in that** the method serves for the manufacture of an artificial hip joint.

13. Artificial joint in accordance with any one of claims 1 to 5, **characterised in that** the artificial joint is an artificial hip joint.

14. Artificial joint in accordance with claim 13, **characterised in that** the shaft of the artificial hip joint has an S-shaped, anatomically matched shaft form with an anteversion in the proximal neck region and with a shaft end projecting towards posterior in a curve or in a kink in order to achieve the longest possible period of anchoring.

15. Artificial joint in accordance with claim 13 or claim 14, **characterised in that** the convex spherical surface B is arranged rotationally symmetrically to a mounting axis D in the direction of the limb neck of the shaft (27) of the artificial hip joint.

**Revendications**

1. Articulation artificielle ayant une coque support (1) qui présente une surface sphérique concave A avec le centre $M_s$ et avec une rotule d'articulation (2) qui présente une surface sphérique convexe B avec le centre $M_k$, où
la coque support (1) et la tête de l'articulation (2) se composent d'un matériau métallique résistant à l'usure,
la surface A présente un rayon moyen $R_m$ et la surface B présente un rayon moyen $r_m$, où leur différence est comprise entre 35 $\mu$m < $R_m$-$r_m$ < 85 $\mu$m,
le défaut de moule de la surface A sur un angle compris entre 90° < $\alpha$ < 180° est inférieur à plus ou moins 7,5 $\mu$m,
le défaut de moule de la surface B sur un angle $\beta$ > 140° est inférieur à plus ou moins 7,5 $\mu$m,
la rotule d'articulation (2) en dehors de la surface B est poursuivie par une surface en retrait C, qui présente une distance plus faible pour aller au centre $M_k$ que la surface B, alors que la rugosité de la surface A correspond à une valeur $R_a$ < 0,08 $\mu$m et la

rugosité de la surface B correspond à une valeur $R_a < 0,08$ µm.

2. Articulation artificielle selon la revendication 1, **caractérisée en ce que** la rugosité de la surface A présente une valeur $HR_a < 0,05$ µm et la rugosité de la surface B présente une valeur $HR_a < 0,05$ µm.

3. Articulation artificielle selon la revendication 1 ou 2, **caractérisée en ce que** la coque support (1) est fermement reliée sur sa face externe à un corps intermédiaire (3) par le biais d'un raccord (7), le corps intermédiaire pouvant être raccordé de manière amovible à une coque externe (4).

4. Articulation artificielle selon la revendication 3, **caractérisée en ce que** la matière du corps intermédiaire est plus de 10 fois élastique par rapport au matériau de la coque support métallique.

5. Articulation artificielle selon la revendication 1, **caractérisée en ce que** la rotule d'articulation peut être reliée par le biais d'un raccord conique amovible (6) dans la direction de l'axe de serrage D avec l'arbre pour obtenir une surface de rotule partielle B ayant le même effet, indépendamment du raccord.

6. Procédé de fabrication d'une articulation artificielle selon la revendication 1, dans lequel la coque support (1), en tant que pièce à usiner, est serrée comme une coque dégrossie au tour avec une masse inférieure dans la zone de la surface support A avec son axe de pole (10) dans l'axe de rotation (14) d'une broche de machine-outil (22) ou la rotule d'articulation (2) dégrossie au tour, en tant que pièce à usiner, est serrée avec une masse supérieure dans la zone de la surface support B avec son axe de serrage D dans l'axe de rotation (15) d'une broche de la machine-outil, et lors de la rotation de la pièce, un corps abrasif cylindrique (17, 18), qui est serré avec son axe cylindrique dans l'axe de rotation (16) d'un porte-outil (19, 20) de manière à être rotatif, est pressé contre la surface support (A, B) de la pièce à usiner (1, 2) avec une arête circulaire (17a, 18) de sa face frontale en ajoutant un liquide abrasif, où l'axe de rotation (16) de la broche porte-outil (19, 20) présente coupe, dans un angle de déviation (γ, δ) < 90°, l'axe de rotation de la poupée porte-pièce (14, 15) en un point d'intersection (25) et où la pression de compression s'exerce en avançant la broche porte-outil (19, 20) dans la direction de l'axe de sa rotation (16).

7. Procédé selon la revendication 6, **caractérisé en ce que** l'angle (δ) entre l'axe de rotation (16) de la broche porte-outil (19) et l'axe de rotation (14) de la broche porte-coque (22) est de 45° et 39° et que le diamètre externe $d_a$ de l'arête circulaire (17a) est choisi de telle sorte que l'inégalité suivante soit respectée :

$$1,6\,R < \frac{d_a}{\cos \delta} < 2,2\,R$$

8. Procédé selon la revendication 6, **caractérisé en ce que** l'angle (γ) entre l'axe de rotation (16) de la broche porte-outil (20) et l'axe de rotation (15) de la broche porte-rotule d'articulation (24) est compris entre 20° et 60° et que le diamètre interne $H_d$ de l'arête circulaire (18i) satisfait à la condition :

$$1,8\,r > H_d > 1,1\,r.$$

9. Procédé selon l'une quelconque des revendications 6 à 8, **caractérisé en ce que** la pièce à usiner comme coque support (1) tourne à une vitesse de rotation $n_s$ ou comme rotule d'articulation (2) tourne à une vitesse de rotation $n_k$, alors que l'outil (17, 18) présente une vitesse de rotation $n_w$ approximativement du double de celle de la pièce à usiner, de préférence, ne forme cependant pas un multiple entier de la vitesse de la pièce à usiner.

10. Procédé selon l'une quelconque des revendications 6 à 9, **caractérisé en ce qu'**il est utilisé pour l'abrasion, pour le pierrage ou pour le polissage.

11. Procédé selon la revendication 8, **caractérisé en ce que**, lors de l'usinage de la rotule d'articulation (2) une mesure du diamètre sur la surface B s'effectue par l'intermédiaire du centre $M_k$ pour déterminer une durée résiduelle pour l'usinage à un diamètre prédéterminé, sur la base des valeurs enregistrées pour la puissance de l'excavation avec une commande.

12. Procédé selon l'une quelconque des revendications 6 à 11, **caractérisé en ce que** le procédé sert à fabriquer une articulation de la hanche artificielle.

13. Articulation artificielle selon l'une quelconque des revendications 1 à 5, **caractérisée en ce que** l'articulation artificielle est une articulation de la hanche artificielle.

14. Articulation artificielle selon la revendication 13, **caractérisée en ce que** l'arbre de l'articulation de la hanche artificielle présente une forme d'arbre en S, adaptée anatomiquement, avec une antéversion dans la zone proximale du col et avec une extrémité de l'arbre située du côté postérieur dans une courbure ou dans un coude, pour arriver à une durée d'ancrage la plus longue possible.

**15.** Articulation artificielle selon la revendication 13 ou 14, **caractérisée en ce que** la surface sphérique convexe B est disposée en une symétrie de rotation par rapport à un axe de serrage D dans la direction du col de l'épaulement de l'arbre (27) de l'articulation de la hanche artificielle.

## Fig. 1

## Fig. 2a

## Fig. 2b

## Fig. 3

EP 0 892 627 B1

**Fig. 4**

**Fig. 5**

12

# Fig. 6